# EUROPEAN PATENT SPECIFICATION

(11) **EP 1 299 559 B1**
(45) Date of publication and mention of the grant of the patent: **28.04.2004**
(21) Application number: 01957896.2
(22) Date of filing: 27.06.2001
(51) Int. Cl.: C12Q 1/25

(54) **METHODS FOR IDENTIFYING INHIBITORS OF THE ANAPHASE PROMOTING COMPLEX**
VERFAHREN ZUR IDENTIFIZIERUNG VON VERBINDUNGEN, DIE DEN ANAPHASE-FÖRDERNDEN KOMPLEX HEMMEN
PROCEDES SERVANT A IDENTIFIER DES INHIBITEURS DU COMPLEXE DE PROMOTION D'ANAPHASE

(30) Priority: 29.06.2000 EP 00113832
(43) Date of publication of application: 09.04.2003
(73) Proprietor: BOEHRINGER INGELHEIM INTERNATIONAL GmbH, 55218 Ingelheim am Rhein (DE)
(72) Inventor: GMACHL, Michael, A-1140 Wien (AT); PETERS, Jan-Michael, A-2100 Korneuburg (AT); GIEFFERS, Christian, A-1020 Wien (AT)
(86) International application number: PCT/EP2001/007329
(87) International publication number: WO 2002/000923

(56) References cited:
- EP-A- 0 843 008
- WO-A-97/37027
- US-A- 5 726 025
- OHTA TOMOHIKO ET AL: "ROC1, a homolog of APC11, represents a family of cullin partners with an associated ubiquitin ligase activity." MOLECULAR CELL, vol. 3, no. 4, April 1999 (1999-04), pages 535-541, XP002154564 ISSN: 1097-2765
- GMACHL MICHAEL ET AL: "The RING-H2 finger protein APC11 and the E2 enzyme UBC4 are sufficient to ubiquitinate substrates of the anaphase-promoting complex." PROCEEDINGS OF THE NATIONAL ACADEMY OF SCIENCES OF THE UNITED STATES, vol. 97, no. 16, 1 August 2000 (2000-08-01), pages 8973-8978, XP002154565 August 1, 2000 ISSN: 0027-8424

## Description

The invention relates to the regulation of the cell cycle in eukaryotic cells. In particular, the invention relates to the key regulator of the cell cycle, the Anaphase Promoting Complex (APC) or cyclosome, as a target for chemotherapeutic drugs.

The aim of most chemotherapeutic approaches against cancer is to kill rapidly proliferating cells while leaving non-proliferating, differentiated cells unaffected. Since the state of the components regulating the cell cycle is different between proliferating and quiescent cells, these components are potential targets for anti-cancer drugs.

The APC is a cell cycle-regulated ubiquitin-protein ligase that regulates important events in mitosis such as the initiation of anaphase and exit from telophase (reviewed by Morgan, 1999; Zachariae, et al., 1999). Together with the ubiquitin-activating enzyme E1 and ubiquitin-conjugating enzymes (E2s) the APC assembles multiubiquitin chains on a variety of regulatory proteins and thus targets them for proteolysis by the 26S proteasome. To initiate sister chromatid separation, the APC has to ubiquitinate the anaphase inhibitor Securin, whereas exit from mitosis requires the APC-mediated ubiquitination of B-type cyclins. These reactions depend on activation of the APC by CDC20 and CDH1, which transiently associate with the APC at the end of mitosis and in G1, respectively. APC substrates contain either a destruction (D) or a KEN box sequence whose presence is required for recognition by the APC (Pfleger and Kirschner, 2000).

In ubiquitination reactions, the APC fulfills the role of a ubiquitin-protein ligase (E3); i.e. it allows the transfer of ubiquitin residues from E2 enzymes to substrate proteins. This reaction results in the formation of an isopeptide bond between the C-terminus of ubiquitin and the epsilon amino group of a lysine residue in the substrate. In subsequent reaction cycles, also lysine residues within the attached ubiquitin residues can serve as acceptor sites, resulting in the assembly of a multiubiquitin chain which is thought to function as a recognition signal for the 26S proteasome. How the APC mediates these ubiquitination reactions is not known. So far nine subunits have been identified in the vertebrate APC, whereas eleven are known in budding yeast (Zachariae, et al., 1999; Peters, 1999). Initial analyses did not reveal any homologies between APC subunits and two previously identified ubiquitin-protein ligases, Ubr1 and E6-AP, suggesting that E3s may represent a structurally and mechanistically diverse class of enzymes. Recent results suggest that the APC belongs to a family of E3s that comprises at least two other multi-subunit complexes, the Skp1-cullin-F box protein complex (SCF) and the von Hippel Lindau (VHL) complex (reviewed in Gieffers, et al., 2000; Deshaies, 1999; Tyers and Jorgensen, 2000). Both the SCF and VHL complex are characterized by the presence of a cullin subunit whose conserved C-terminal domain interacts with a protein called Rbx1/Hrt1/Roc1 in budding yeast and ROC1 in humans (Ohta, et al., 1999; Skowyra, et al., 1999; Tan, et al., 1999; Seol, et al., 1999; Kamura et al., 1999). This protein may recruit the E2 enzyme CDC34 to the SCF and is characterized by the presence of a RING-H2 finger consensus, a subtype of the RING finger motif, which is known to coordinate two atoms of zinc (Borden and Freemont, 1996). The APC subunit APC2 is a distant member of the cullin family (Zachariae, et al., 1998; Yu, et al., 1998), and a RING-H2 finger protein related to Rbx1/Hrt1/Roc1, called Apc11, has been identified in the budding yeast APC (Zachariae, et al., 1998).

Although various functions have been proposed for RING finger motifs such as representing nucleic acid or protein binding domains, numerous recent observations suggest a prominent role of these domains in ubiquitination reactions. The analysis of rbx1/hrt1/roc1 and apc11 mutants in budding yeast indicates that these proteins are essential for SCF and APC activity, respectively (Ohta, et al., 1999; Skowyra, et al., 1999; Seol, et al., 1999; Kamura et al., 1999; Zachariae, et al., 1998). A dimeric complex of CUL1 and ROC1 obtained by transient transfection into human cells and partial purification has been reported to catalyze the assembly of unanchored multiubiquitin chains in the presence of E1 and CDC34, suggesting that a cullin and a RING-H2 finger subunit may represent the essential catalytic subunits of this type of E3 (Tan, et al., 1999). Also immunoprecipitates from transiently transfected cells containing APC11, APC2 and some unidentified proteins have been reported to contain ubiquitination activity (Ohta, et al., 1999). Intriguingly, RING-H2 finger domains have also been identified in several E3s that do not contain cullin subunits. For example, RING-H2 finger sequences are required for the activity of Ubr1, an E3 that ubiquitinates proteins with destabilizing N-terminal amino acid residues (Xie and Varshavsky, 1999), for the activity of MDM2, a protein that can ubiquitinate the tumor suppressor p53 (Honda and Yasuda, 2000; Fang, et al., 2000), and for the activity of c-CBL, a protein that can mediate the ubiquitination of the PDGF receptor (Yokouchi, et al., 1999; Joazeiro, et al., 1999; Levkowitz, et al., 1999). When expressed in *E. coli*, several other RING-H2 finger proteins have been shown to support the synthesis of multiubiquitin chains, suggesting that many if not all proteins with this motif may have this ability (Lorick, et al., 1999).

The APC has been suggested as a target for chemotherapeutic intervention for the following reasons:
1. The activity of the APC is essential for sister chromatid separation, for the function of the mitotic spindle and for exit from mitosis during cell proliferation. Interfering with this function would prevent tumor cells from completing mitosis.
2. Most tumor cells have highly abnormal karyotypes. They undergo anaphase in the presence of chromosomal damage that would prevent activation of the APC in normal cells. Tumor cells might therefore be especially sensitive to drugs that interfere with APC function.

In WO 96/33286, it has been suggested to identify APC inhibitors by incubating the mitotic destruction complex, APC, in a reaction containing a ubiquitin activating enzyme (E1), a suitable ubiquitin conjugating enzyme (E2), ubiquitin, ATP and a substrate (e. g. cyclin B) which can be detected either by immunoblotting or because it is labeled radioactively, and determining whether a test compound inhibits the ubiquitination reaction.

WO 98/21326 describes a method for identifying the APC subunits and using these subunits to provide APC in recombinant form to be employed in an assay to identify APC inhibitors.

Ubiquitination reactions are characterized by the attachment of the free carboxy group of activated ubiquitin to an epsilon amino group of a lysine residue within a given protein. After attachment of the first ubiquitin residue, lysine residues within this ubiquitin molecule are used for further attachment of ubiquitin molecules by the same mechanism, leading to multiubiquitinated substrate proteins.

As ubiquitin contains more than one lysine, the multiubiquitin chain is usually branched. The attachment of ubiquitin to a given substrate protein therefore leads to a dramatic change in the molecular weight of the thus modified substrate protein.

It was an object of the invention to further elucidate the mechanism of the APC's ubiquitination activity, in particular to identify the subunit(s) responsible for this reaction in order to provide an assay useful for identifying APC inhibitors.

It was surprisingly found that APC11, which is a small 10 kDa RING finger protein and represents only about 1 % of the total APC mass, is required as the only APC component to allow the synthesis of multiubiquitin chains in the presence of E1 and E2. Due to the physiological role of the APC subunit APC11 to be the APC subunit that is relevant for the APC's function as a ubiquitin-protein ligase, assays may be set up that use APC11 in the place of the holo APC, which greatly simplifies the search for APC inhibitors.

In the experiments of the present invention, the change in molecular weight of the ubiquinated APC11 was visualized by separating the products of an ubiquitination reaction on SDS-PAGE followed by Coomassie Blue staining or transfer to PVDF or nitrocellulose membranes, where either the substrate protein or ubiquitin can be monitored using adequate antibodies. These multiubiquitin chains were detected in reaction mixtures comprising recombinant E1 and E2, an ATP regenerating system and ubiquitin in the presence of GST-tagged APC11, that had been obtained by expression in *E. coli* DH5α cells. Upon incubation at 37°C, but not upon incubation on ice, the formation of multiubiquitin chains was detectable.

The specificity of the observed reaction was tested by introducing recombinant APC subunits other than APC11. To this end, GST fusions with human APC2, APC5, APC7, APC8/CDC23 (Yu, et al., 1998), APC3/CDC27 (Tugendreich, 1993), APC6/CDC16 (Tugendreich, et al., 1995), and APC10/DOC1 (Grossberger, et al., 1999) were constructed and expressed in *E. coli* DH5α cells. The formation of multiubiquitin chains was only detected in samples containing GST-APC11.

Further analyses of the reaction products of an APC11-mediated ubiquitination reaction revealed that the multiubiquitin chains that are formed in the presence of GST-APC11 are in part free ubiquitin chains which are not attached to any protein, and in part attached to GST-APC11 itself. This autoubiquitination of GST-APC11 leads to a complete disappearance of the unmodified GST-APC11 and conversion into high molecular weight forms upon incubation for 30 min at 37°C under standard conditions, i.e. in the presence of 0.5µg E1, 0.5µg E2, 1µg ubiquitin, ATP and 0.5µg GST-APC11.

Introduction of APC specific substrates, like CyclinB or Securin, both proteins tagged at the carboxy-terminus with a myc-epitope for antibody detection and a His(6) tag for affinity purification, lead to the conversion of these proteins into multiubiquitinated forms as detected by immunodecoration with antibodies against the myc-epitope (9E10). Control reactions containing unrelated proteins, like maltose binding protein or GST-p53, were not ubiquitinated under the same conditions.

The present invention relates to a method for identifying a compound that inhibits the ubiquitination reaction mediated by the APC, characterized in that a compound's ability to interfere with the formation of multiubiquitin chains by APC11 is determined.

In an embodiment, the present invention relates to a method for identifying compounds that inhibit the ubiquitination reaction mediated by the APC, characterized in that APC11 is incubated, together with a ubiquitin activating enzyme (E1), a ubiquitin conjugating enzyme (E2), ubiquitin and ATP for a period of time sufficient to obtain a measurable level of ubiquitination of APC11, and comparing the level of ubiquitination of APC11 in the presence or absence of a test compound.

This embodiment is based on the principle of self-ubiquitination of APC11.

The components for this type of assay can be obtained as follows:

The proteins used in the screening assay are preferably recombinant proteins, which can be obtained according to conventional methods by transforming a suitable host with a plasmid carrying the sequence encoding the protein. In the case of APC11, the preferred host cells are *E. coli* cells, because they are devoid of endogenous APC. The cDNA sequences of the protein components APC11, E1, E2 and ubiquitin are available from the literature and from databases. The assay components APC11, preferably human APC11 (Gene Bank Accession No. AF247565), E2 and optionally ubiquitin are usually produced and purified as fusion proteins; E1 is purified due to its reversible interaction with ubiquitin according to known methods (e.g. Hatfield, et al., 1990; Hatfield and Vierstra, 1992). In the case of using untagged ubiquitin, this assay component is commercially available (e.g. from Sigma, Fluka).

Preferably, the naturally occurring proteins used; however, the proteins may contain deviations from the natural amino acid sequence as long as these deviations do not impair their functional activity. The proteins may be fused to an affinity tag, which is a protein suitable for affinity purification, such as gluthathion S-transferase (GST, Amersham Pharmacia), maltose binding protein (MBP, New England Biolabs) or short tags like His(6) (Qiagen). The obtained fusion protein can be expressed, e.g. in *E. coli*, and purified according to standard protocols.

A suitable ubiquitin activating enzyme is the wheat UBA1 E1 (gene bank Accession number M55604), however, UBA1 E1 from other species may also be used, which can be purified on a ubiquitin affinity matrix according to published procedures (e.g. Hatfield, et al., 1990; Hatfield and Vierstra, 1992).

As ubiquitin conjugating enzyme (E2) in a preferred embodiment, the human variant UBCH5b (gene bank accession number U39317) is used, although, also in this case, UBCH5b homologues from other species, e.g. Xenopus laevis, may be employed. Alternatively, UBCH5a or UBCH5c can be used. Preferably the ubiquitin conjugating enzyme E2 is fused to an affinity tag which is selected from the ones listed above as suitable for APC11, but different from the tag chosen for APC. For example, in the case that GST-APC11 is used, His(6) or another tag different from GST is used for tagging E2.

To sustain a sufficient ATP level during the entire ubiquitination reaction, a so-called "ATP regenerating system" may be advantageously employed (Murray, 1991).

Ubiquitin is commercially available (Sigma), it may also be recombinantly produced; in this case it may be fused to various tags for purification, i.e. His(6), GST or for detection, i.e. myc-epitope, HA-epitope. In both cases, ubiquitin comprises the N-terminal 76 amino acids required for its function. Preferably, a tagged ubiquitin is employed in the assay. The ubiquitin used in the assay may also carry a non-proteinacious tag, e.g. biotin.

The above-described assay essentially comprises the steps of the ubiquitination reaction itself and the step of measuring the extent of ubiquitination on APC11.

The first step comprises reacting the assay compounds listed above for a period of time sufficient to allow for the ubiquitination reaction, e.g. for 30 min.

The reaction may either be conducted in solution by simply mixing the assay components or alternatively, the reaction may take place by using immobilized APC11. In this case, APC11 carries an affinity tag (GST or one of the alternative tags mentioned above) that is used for its binding to a solid phase carrying the ligand for the respective affinity moiety, e.g. glutathione agarose or sepharose beads or microtiter plates coated with antibodies against the affinity moiety, e.g. commercially available anti-GST antibodies.

After the reaction has been completed, the extent of multiubiquitin chain formation, i.e. the covalent association of ubiquitin with APC11, can be measured in different ways:
In case the ubiquitination reaction has been conducted in solution, the affinity-tagged, e.g. GST-tagged, APC11 is captured on microtiter plates that are coated with an antibody against GST (this step can be omitted in case the reaction has been carried out with APC11 bound to a solid phase). The unbound GST-APC11, the unincorporated ubiquitin and the other reaction partners are then washed away. Subsequently the immobilized ubiquitin can be visualized by using an antibody that is directed against an epitope, e.g. the myc-epitope present in the recombinant ubiquitin, which antibody carries a detectable label. Suitable labels are radioactive labels, e.g. ¹²⁵I, enzymatic labels, e.g. horseradish peroxidase or alkaline phosphatase, or fluorometric labels. In a preferred embodiment, quenched fluorophors, e.g. Europium (Wallac, Turku, Finland) that will be dequenched upon incubation with an enhancer solution (Wallac), are used. The obtained values are compared to values obtained from reactions without APC11 (negative control, background) and to a reaction, incubated with the solvent DMSO only (positive control).

Alternatively to using the ELISA type assay described above to detect the amount of bound ubiquitin, the physical proximity of ubiquitin molecules to APC11 (in the case of measuring self-ubiqutitination of APC11), to the APC substrate (in the case of determining ubiquitination of an APC substrate; see below) or to itself (in the case of determining the formation of multiubiquitin chains independent of whether they are attached to APC11 or not, see below), upon incubation at 37°C can be used to measure the extent of ubiquitin incorporation into high molecular forms by fluorescence resonance energy transfer (FRET, as described by Gershkovich et al., 1996, or by Matayoshi et al., 1990, ore recently reviewed by Selvin, 2000). FRET can only be achieved if certain conditions are fulfilled, i.e. fluorophor pairs with overlapping emission and excitation wavelengths, like europium/allophycocyanin, europium/Cy5, europium/PE (all commercially available from Wallac, Turku, Finland) and an minimal proximity of these fluorophors below 5-10nM. These fluorophors can be added either bound to antibodies directed against the affinity label, e.g. GST, or the epitope, e.g. the myc epitope, or can be directly coupled to APC11 or ubiquitin (custom service of Wallac). When coupled to antibodies, the fluorophors are added to the reaction after its completion. No further washing steps are necessary and signals (excitation at 340 nm and emission measurement at 665 nm in the case of the FRET pair allophycocyanin and europium) are measured after incubation at 4°C for 30 min, allowing the binding of the antibodies and the subsequent energy transfer between the fluorophors. In case of direct labeling of reaction components, i.e. ubiquitin or APC11, real time measurements can be performed allowing the detection of kinetic differences in the reaction.

In another embodiment, the present invention relates to a method for identifying compounds that inhibit the ubiquitination reaction mediated by the APC, characterized in that APC11 is incubated, together with a ubiquitin activating enzyme (E1), a ubiquitin conjugating enzyme (E2), ubiquitin and ATP for a period of time sufficient to obtain a measurable level of multiubiquitin chains, and comparing the level of multiubiquitin chains in the presence or absence of a test compound.

This embodiment is based on the detection of multiubiquitin chains that are formed during the course of the reaction, independent of whether they are attached to APC11 or not. In this embodiment, besides unattached multiubiquitin chains, APC11-bound multiubiquitin chains are also detected.

Preferably, this variant of the assay uses the FRET technology as described above. Such an assay can be conducted by either introducing two differently tagged versions of ubiquitin, i.e. a combination of a myc-tagged ubiquitin containing 3, 6 or 9 myc-epitopes with a GST-tagged ubiquitin, or a combination of a myc-tagged ubiquitin with a biotinylated ubiquitin; or by directly labeling ubiquitin with two different fluorophors. Ubiquitination reactions can then be carried out as described above, but in the presence of equal amounts of differently tagged or labeled ubiquitin moieties. Covalent incorporation of these ubiquitin molecules into multimers allows the generation of a FRET signal. The change of such a FRET signal upon inclusion of compounds is used as read out in identifying inhibitors.

As a negative control, the reaction is conducted in the absence of APC11.

In an alternative embodiment, the invention relates to a method for identifying compounds that inhibit the ubiquitination reaction mediated by the APC, characterized in that APC11 is incubated, together with a ubiquitin activating enzyme (E1), a ubiquitin conjugating enzyme (E2), ubiquitin, ATP and an APC substrate protein for a period of time sufficient to obtain a measurable level of ubiquitination of the substrate protein and comparing the level of ubiquitination of the APC substrate protein in the presence or absence of a test compound.

This embodiment is based on the observation that APC11 alone is able to ubiquitinate substrate proteins of native APC, but not any other protein, e.g. maltose binding protein MBP, and not or only weakly ubiquitinates proteins that can be otherwise ubiquitinated in vivo (p53).

Examples for APC substrates are CyclinB or Securin, which are preferably employed, like the other reaction partners, in recombinant form (Pines and Hunter, 1989; Zou, et al., 1999).

With regard to the assay components and the reaction conditions, the considerations described for the first embodiment, i.e. the assay based on APC11 autoubiquitination, apply also for the other two embodiments, i.e. the assay based on formation of multiubiquitin chains independent of their attachment to APC11 and to the assay based on substrate ubiquitination.

Preferably, the assay methods of the invention are conducted in the high throughput format. By way of example, such an assay is performed in 96 well plates in a suitable reaction volume, e.g. 50 µl, in the absence or presence of the test compounds, which are usually dissolved in DMSO.

In the embodiment that uses APC substrate proteins, these are captured by virtue of a tag, preferably myc, and the amount of associated ubiquitin is quantified in the presence or absence of test compounds.

Compounds identified as positive hits in the above-described primary screening assays have to be confirmed to be specific inhibitors of APC11 activity and not to be inhibitors of the other enzymes present in the reaction mixture.

Possible candidates for unspecific hits are inhibitors of ubiquitin activating enzyme (E1) and ubiquitin conjugating enzyme (E2). E1 is necessary to activate ubiquitin for the subsequent reaction, as it is the only enzyme that is able to recognize free ubiquitin in solution. E1 forms a thioester with ubiquitin in an ATP-dependent manner and transfers this now activated ubiquitin molecule to an E2 enzyme, where another thioester between E2 and ubiquitin is formed. The thioester formation of E2 with ubiquitin is strictly dependent on the presence of E1, meaning that E2 alone is unable to form a thioester with free ubiquitin. Compounds that inhibit any step in this cascade, i.e. substances that interfere with the ATP binding to E1 or that prevent the formation of the thioesters between ubiquitin and E1 or ubiquitin and E2 like any reducing agent will subsequently inhibit the formation of multiubiquitin chains and appear as positive hits. The ATP dependent thioester formation can be used to identify all compounds that inhibit this first step in the formation of multiubiquitin chains. Assays that are able to measure thioester formation are known in the literature (Yu, et al., 1996). In brief, they are based on using the nature of a thioester concerning reducibility through agents like dithiothreitol or β-mercapto ethanol. A mixture of E1, E2, ATP and ubiquitin is incubated at 37°C in the presence of the compounds identified to inhibit the formation of multiubiquitin chains dependent on GST-APC11 or DMSO as control. These samples are subsequently subjected to SDS-PAGE under non-reducing conditions; i.e. thioesters that have formed will not be broken up and transferred to PVDF or nitrocellulose membranes. Ubiquitin can be detected either by using monoclonal antibodies against ubiquitin (commercially available, e.g. from Santa Cruz) or by using and detecting tagged versions of ubiquitin (GST-ubiquitin, anti-GST antibodies; 3-9 x myc-ubiquitin, 9E10 antibodies). In control reactions, free ubiquitin can be detected as well as ubiquitin associated with an approx. 20 kDa protein (E2) and an approximately 120 kDa protein (E1). Reduction or disappearance of these thioesters upon incubation with inhibitory compounds can be taken as indication for inactivation of either E1 or E2 or both and considered as unspecific.

Advantageously, a further secondary assay is employed to confirm that the compounds identified in the assays of the invention are inhibitors specific for a APC11-mediated ubiquitination reaction and not inhibitors of reactions mediated by other RING-finger containing proteins. Such an assay uses another RING-finger containing protein, e.g. MDM2 (Gene Bank Accession No. U33199), ROC1/Hrt1/Rbx1 (Gene Bank Accession No. AF142059) or ROC2 (Gene Bank Accession No. AF142060).

MDM2 is described in the literature as a protein responsible for binding and ubiquitinating the tumor suppressor protein p53. This reaction can be performed in vitro using recombinant proteins. During the course of the reaction, MDM2 is autoubiquitinated in a similar manner as APC11. Moreover, this reaction is dependent on the very same auxiliary proteins, i.e. E1 and E2, in particular UBCH5b. MDM2-dependent autoubiquitination can therefore be used either in a parallel screen using the very same components as described for the APC11-dependent reaction (except for APC11) or to evaluate, in a subsequent step, the compounds identified in the APC11-dependent screen. Any compound that selectively inhibits one of the two reactions will be considered as specific for APC11 or MDM2, respectively. (In the case of employing specificity controls to exclude ROC1 or ROC2 inhibitors, the assay is performed in an analogous manner, using, instead of MDM2, recombinant ROC1 or ROC2, respectively.)

As a parallel or secondary screen for compounds identified in the assay employing an APC substrate, the above-described MDM2-dependent ubiquitination of p53 can be used as a specificity control. Compounds that specifically inhibit either MDM2-dependent p53 ubiquitination or APC11 dependent Securin or CyclinB ubiquitination are considered specific for the respective reaction.

As pointed out above, the naturally occurring APC (holo APC) is a multisubunit protein complex which is specifically regulated during the cell cycle by virtue of the interaction with other proteins like CDC20, CDH1, unidentified kinases and phosphatases. To confirm that a compound that has been identified in the above-described primary and optionally secondary screen as an inhibitor of APC11 ubiquitination activity also inhibits halo APC and is therefore a drug candidate which is capable of inhibiting the cell's entry into the subsequent cell cycle, the compound is subjected to a secondary screen employing holo APC.

In such a secondary/tertiary screen, an APC with the ability of the native APC to ubiquitinate APC substrate protein in a strictly regulated manner (the regulation depending inter alia, on binding to regulatory proteins like CDH1 or CDC20 and on the phosphorylation status) is employed, e.g. immunoprecipitated APC, preferably from human cells, in particular from HeLa. Alternatively, recombinant APC may be used which can be produced e.g. according to the method described in WO 98/21326. This APC may be immobilized on a solid support, preferably beads, covalently associated with antibodies against an APC subunit, e.g. APC3/CDC27 (Kramer et al., 1998); in the case of recombinant APC, it may be present in solution. In a second step, E1, E2, in particular UBCH5b or UBCH10, or a combination of both, ubiquitin, either radioactively labeled for direct detection of multiubiquitin chains or unlabeled for detection of substrate conjugates, an ATP regenerating system and substrate proteins such as CyclinB or Securin, preferably from human source, are added in the presence or absence of test compounds. The substrate proteins are either labeled with ¹²⁵I or detectable through the addition of a carboxy-terminal myc tag recognized by the 9E10 antibody followed by a His(6) tag for affinity purification. Reactions can either be analysed by SDS-PAGE following Phosphorimaging (when using iodinated proteins) or Western blotting using appropriate antibodies (9E10, anti-ubiquitin). Alternatively, this assay can also be performed on microtiter plates covered with antibodies against GST. Substrate proteins like CyclinB or Securin can be fused to GST and can therefore be captured on the plates after the reaction is completed. The amount of associated ubiquitin can be measured either directly, when using ¹²⁵I-ubiquitin or quantified using antibodies against ubiquitin, or against a tag associated with ubiquitin. These antibodies are either directly labeled, i.e. with a fluorophore or with ¹²⁵I, or will be detected with a secondary, labeled antibody.

Compounds identified in the screening methods of the invention, which function as APC inhibitors, are expected to arrest cells in metaphase of mitosis; this arrest is expected to subsequently induce apoptotic cell death. Due to this ability, such compounds are drug candidates for the therapy of cancer.

To test the ability of a substance to inhibit tumor cell proliferation, primary human tumor cells are incubated with the compound identified in the screen and the inhibition of tumor cell proliferation is tested by conventional methods, e.g. bromo-desoxyuridine or ³H incorporation. Compounds that exhibit an anti-proliferative effect in these assays may be further tested in tumor animal models and used for the therapy of tumors.

Toxicity and therapeutic efficacy of the compounds identified as drug candidates can be determined by standard pharmaceutical procedures, which include conducting cell culture and animal experiments to determine the IC₅₀, LD₅₀, ED₅₀. The data obtained are used for determining the human dose range, which will also depend on the dosage form (tablets, capsules, aerosol sprays, ampules, etc.) and the administration route (oral, buccal, nasal, paterental or rectal). A pharmaceutical composition containing the compound as the active ingredient can be formulated in conventional manner using one or more physiologically active carriers and excipients. Methods for making such formulations can be found in manuals, e.g. "Remington Pharmaceutical Sciences".

### Brief description of the figures:

- Fig. 1:: The APC subunit APC11 is able to catalyze the formation of multiubiquitin chains
- Fig. 2:: Characterization of ubiquitin conjugates formed in the presence of GST-APC11
- Fig. 3:: Ubiquitination reactions occurring in the presence of APC11 are dependent on E1, UBC4, ATP and ubiquitin
- Fig. 4:: The formation of multi-ubiquitin chains depends on the integrity of the APC11 RING-H2 finger
- Fig.5:: GST-APC11 mediates the ubiquitination of the APC substrate Securin
- Fig. 6:: Kinetic analysis of GST-APC11-mediated formation of multiubiquitin chains measured by FRET
- Fig. 7:: Inhibition of APC11-mediated formation of multiubiquitin chains exemplified by the reducing agent DTT

If not otherwise stated, the following materials and methods were used in the experiments of the present invention:

### (a) DNA constructs

Fusion proteins between glutathione-S-transferase (GST) and APC2, APC3/CDC27, APC5, APC6/CDC16, APC7, APC8/CDC23, APC10/DOC1, CDC26 and APC11 were generated by polymerase chain reaction (PCR) based amplification of individual open reading frames, starting from amino acid 2 until the stop codon and subsequent blunt end ligation into the pGex4T-1 plasmid (Pharmacia) cut with BamH1 and filled in with T4 DNA-polymerase. The APC2, APC3/CDC27, APC5, APC6/CDC26, APC7, APC8/CDC23 and APC10/DOC1 coding region were amplified from cDNA containing plasmids, whereas APC11 and CDC26 were amplified from single stranded cDNA prepared from 3 µg total HeLa RNA. Human Securin was amplified from HeLa single stranded cDNA and cloned into pTrcHis2A (Invitrogen) creating a fusion with a c-terminal myc-His tag. Mutagenesis of Cys23, Cys26, Cys51 and Cys54 of APC11 and Arg61 and Leu64 of human Securin to alanine was performed using standard PCR based technology. All mutations were evaluated by DNA sequencing. His-tagged UBCH5b was generated by recloning the open reading frame into the pQE30 vector (Qiagen).

### b) Protein expression and purification

*E. coli* DH5α carrying the individual expression plasmids were grown over night, induced with 0.3 mM isopropyl-b-thiogalactopyranoside for 2 to 3 hours and harvested by centrifugation. Lysis was performed either under native (GST-APC2, GST-APC3, GST-APC5, GST-APC6, GST-APC7, GST-APC8, GST-APC10, GST-CDC26 and GST-APC11 wildtype and mutants, His-UBC4) or denaturing conditions (Securin-myc-His and Securin mutant). In brief, Securin was purified on nickel-nitrilotriacetic acid agarose (Qiagen) using 8 M urea as denaturing agent. Subsequently the eluted protein was dialyzed against buffer QA (Kramer, et al., 1998) containing decreasing amounts of urea. Purification of GST-fusion proteins was performed by lysis in Tris-KCI (TK) buffer (50 mM Tris pH 8.0, 150 mM KCl), plus 2 mM DTT and 1% Triton X-100 followed by affinity purification on glutathione sepharose (Pharmacia). Bound proteins were eluted in TK, 2 mM DTT and 10 mM glutathione. Free glutathione was removed by repeated concentration and redilution in TK, 0.5 mM DTT using Centriprep 10 concentrators (Amicon). In some experiments, GST-tagged APC subunits bound to glutathione beads were used instead of soluble, eluted proteins. His-UBC4 was purified on nickel-nitrilotriacetic acid agarose in TK, 1 mM fl-mercaptoethanol (b-ME), 1% Triton X-100. Protein was eluted in TK, 1 mM b-ME, 250 mM imidazole and concentrated/rediluted on Centriprep10 using TK, 1 mM b-ME. UBA1 (E1) and UBCx were expressed and purified as described (Yu, H. et al., 1996). UBC2 was a gift from M. Rolffe, Cambridge, MA. CDC34 was a gift from R. Yew, Boston, MA. APC was immunopurified from HeLa log phase cells using a polyclonal anti-CDC27 antibody (Kramer, E. et al., 1998).

### c) Ubiquitination assays

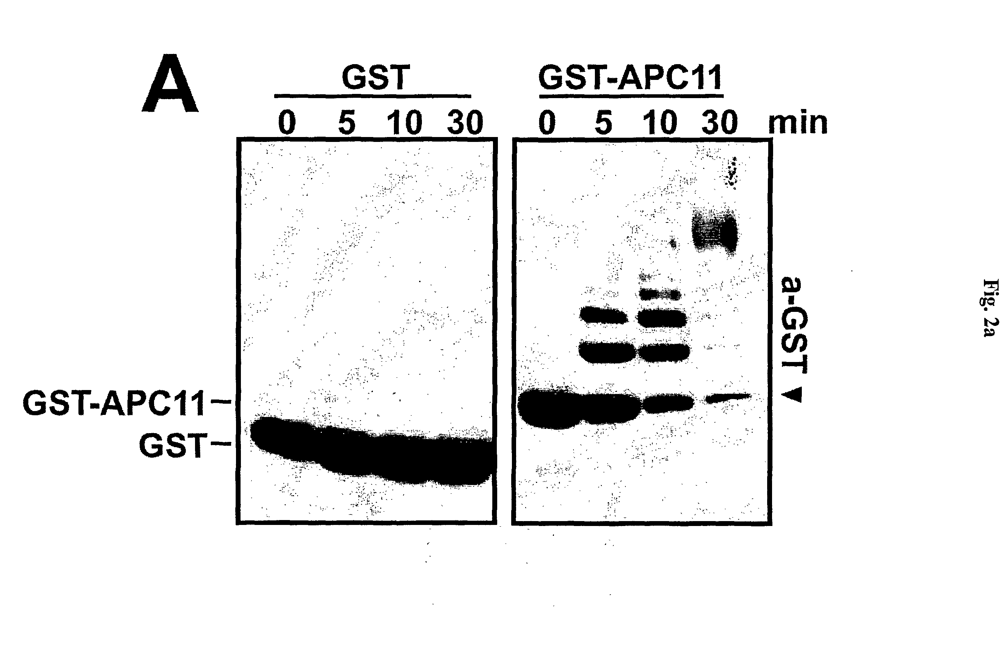

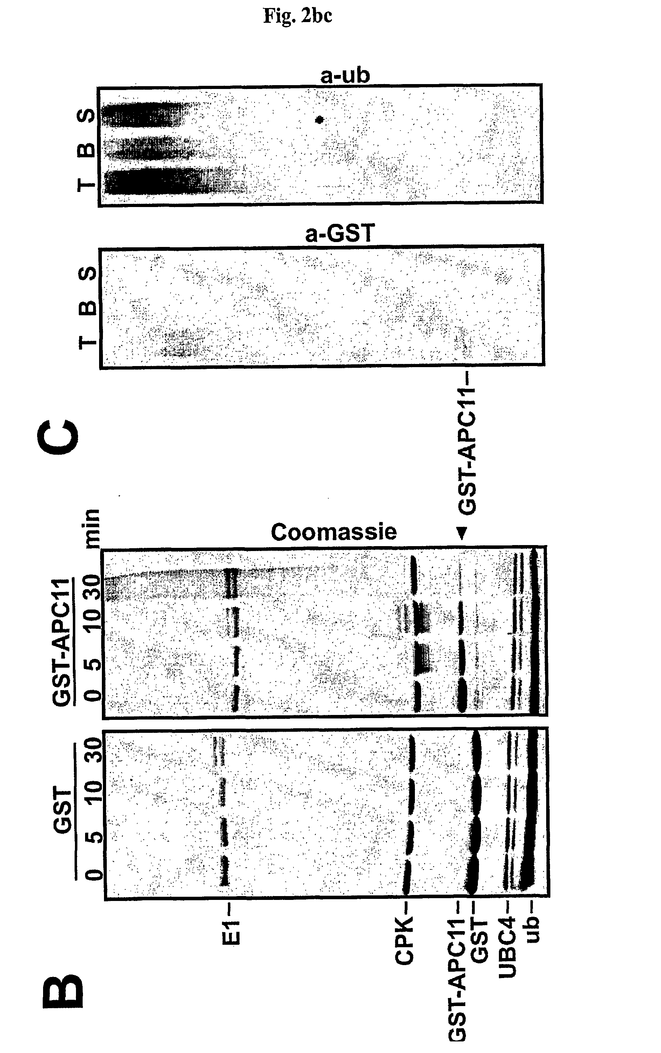

Standard ubiquitination reactions were carried out in 10 µl QA for various time periods and contained 0.5 µg GST-fusion protein, either bound to glutathione-sepharose and washed with QA or added as purified, soluble protein, 0.5 µg E1, 0.5 µg E2, 20 µg ubiquitin or where indicated iodinated ubiquitin, 0.5 µl of an ATP regenerating system and where indicated 20 or 50 ng of substrate (Securin-myc-His, maltose binding protein (MBP), GST-p53). Reactions were stopped by addition of sodium-dodecyl-sulfate (SDS) containing sample buffer. Ubiquitination assays in Fig. 2C were incubated for 30 min at 37°C and diluted 20 fold with cold QA containing 0.5% Triton X-100. After addition of 5 µl glutathione agarose, the reaction was incubated for 60 min at 4°C with agitation. Before centrifugation an aliquot of the reaction was removed (total, Fig. 2C). A second aliquot was removed after pelleting the agarose beads (supernatant, Fig. 2C). The remaining bead fraction was washed twice with QA, 0.5% Triton X-100 and a final aliquot was removed (beads, Fig. 2C). Thioester formation experiments were performed as described (Yu, et al, 1996).

### d) Antibodies

Rabbit polyclonal antibodies against CDC26 were generated with a peptide encoding 35 N-terminal amino acids. Antibodies against GST, MBP, ubiquitin and p53 were obtained from Santa Cruz Biotechnology. All other antibodies used have been described (Yu, et al, 1996).

### Example 1

The APC subunit APC11 is able to catalyze the formation of multiubiquitin chains.

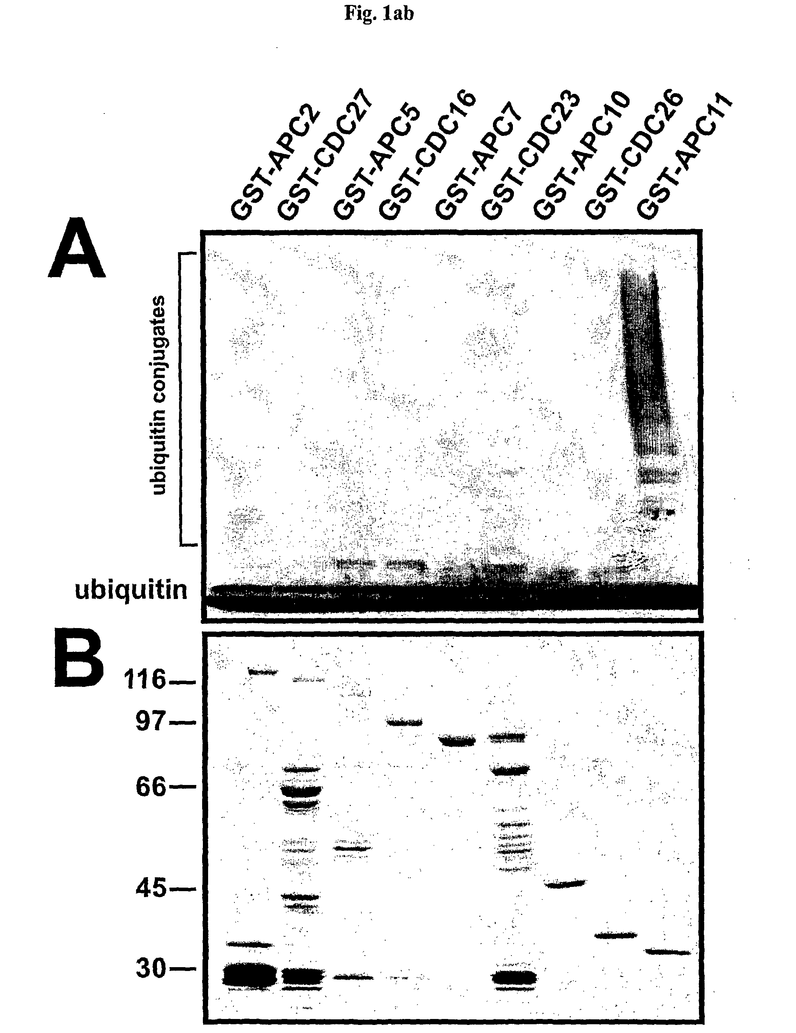

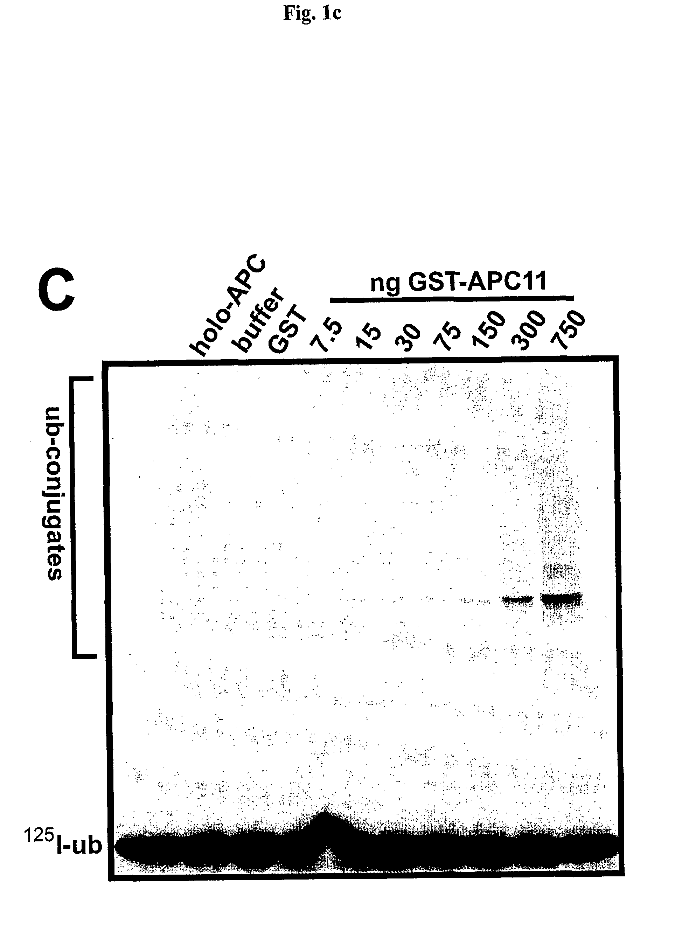

The identification of CDC26 and APC11 increased the number of known human APC subunits to eleven, but for none of these it is known how they function in ubiquitination reactions. To address this question nine human APC subunits were expressed as recombinant proteins containing an N-terminal glutathione S transferase (GST) tag (Fig. 1A). To avoid possible heterooligomerization problems with endogenous APC subunits all proteins were expressed in *E. coli* whose genome does not contain obvious APC homologs. To analyze their activities, the purified GST-fusion proteins were incubated with E1, UBC4, ubiquitin and an ATP-regenerating system and the formation of multiubiquitin chains were measured by immunoblotting with ubiquitin antibodies. Remarkably, under these conditions multiubiquitin chains were formed in the presence of GST-APC11 but not in the presence of any of the other APC subunits (Fig. 1B). In dose response experiments the amount of ubiquitin conjugates formed was dependent on the concentration of GST-APC11 (Fig. 1C). Multiubiquitin chains could also be detected when radiolabeled ubiquitin (Fig. 1C), GST- or myc-tagged ubiquitin were used (data not shown). Interestingly, similar multiubiquitin chains were generated by purified human APC under these conditions, i.e., in the absence of substrates (Fig. 1C, holo-APC).

Fig. 1: (A) Bacterial lysates containing GST-fusions to APC2, APC3,

APC5,APC6, APC7, APC8, APC10, CDC26 and APC11 were incubated with glutathione beads, washed, separated by SDS-PAGE and stained with Coomassie Blue. (B) Identical amounts of lysates as in (A) were bound to glutathione beads, washed and used for in vitro ubiquitination.(C) Purified holo-APC, buffer QA, GST (750 ng) or the indicated amounts of GST-APC11 were incubated with E1, UBC4 and ATP as in (B) in the presence of ¹²⁵I-ubiquitin. Samples were incubated for 10 min at 37°C, separated by SDS-PAGE and visualized by phosphorimaging.

### Example 2

### Characterization of ubiquitin conjugates formed in the presence of GST-APC11

When the behaviour of GST-APC11 and UBC4 in these reactions was analyzed by immunoblotting it was observed that GST-APC11 itself was quantitatively converted into multiubiquitinated forms (Fig. 2A), whereas UBC4 was only monoubiquitinated (data not shown). It was possible to detect the formation of multiubiquitin chains also by Coomassie blue staining (Fig. 2B). A comparison of the Coomassie stained conjugates with the GST-APC11 containing conjugates detected by immunoblotting suggested that only a portion of the multiubiquitin chains are bound to GST APC11. To test this possibility, GST-APC11 was removed at the end of the reaction by the addition of glutathione beads and centrifugation. Quantitative detection of the conjugates with radiolabeled ubiquitin antibodies showed that approximately 60% of these chains remained in the supernatant, although GST-APC11 was exclusively detected in the pellet (Fig. 2C), demonstrating that most multiubiquitin chains are not bound to GST-APC11. Due to the lack of suitable antibodies it was impossible to test directly whether these chains are anchored to E1 or the creatine phosphokinase which is present in the ATP-regenerating system. However, this is unlikely because Coomassie staining indicated that creatine phosphokinase is not detectabely modified during the course of the reaction, whereas about 50 % of the E1 in the reaction mixture is converted into a discrete ubiquitinated form (Fig. 2B). Analysis of this E1 modification using GST-ubiquitin suggested that E1 is diubiquitinated under these conditions (data not shown).

Fig. 2: (A) Purified GST (left) or GST-APC11 (right) were incubated for the indicated time with E1, UBC4, an ATP regenerating system and ubiquitin as in Fig 1 B. Samples were separated by 12% SDS-PAGE. GST and GST-APC11 were detected by immunodecoration with anti GST antibodies. The position of unmodified GST-APC11 is indicated by an arrowhead. (B) Identical samples as in (A) were separated by 15 + 8% SDS-PAGE and stained with Coomassie Blue. Identified proteins are indicated on the left (CPK, creatine phosphokinase, all others see text). The arrowhead indicates the position of GST-APC11. (C) A GST-APC11-mediated ubiquitination reaction as described in Fig. 2B was split into three aliquots as described in materials and methods. Identical amounts of total (T), beads (B) and supernatant (S) fractions were loaded on a 15 + 8% polyacrylamid gel and transferred to PVDF membrane. One part (left) was decorated with anti-GST antibodies, the other part (right) with anti-ubiquitin antibodies.

### Example 3

Ubiquitination reactions occurring in the presence of APC11 are dependent on E1, UBC4, ATP and ubiquitin.

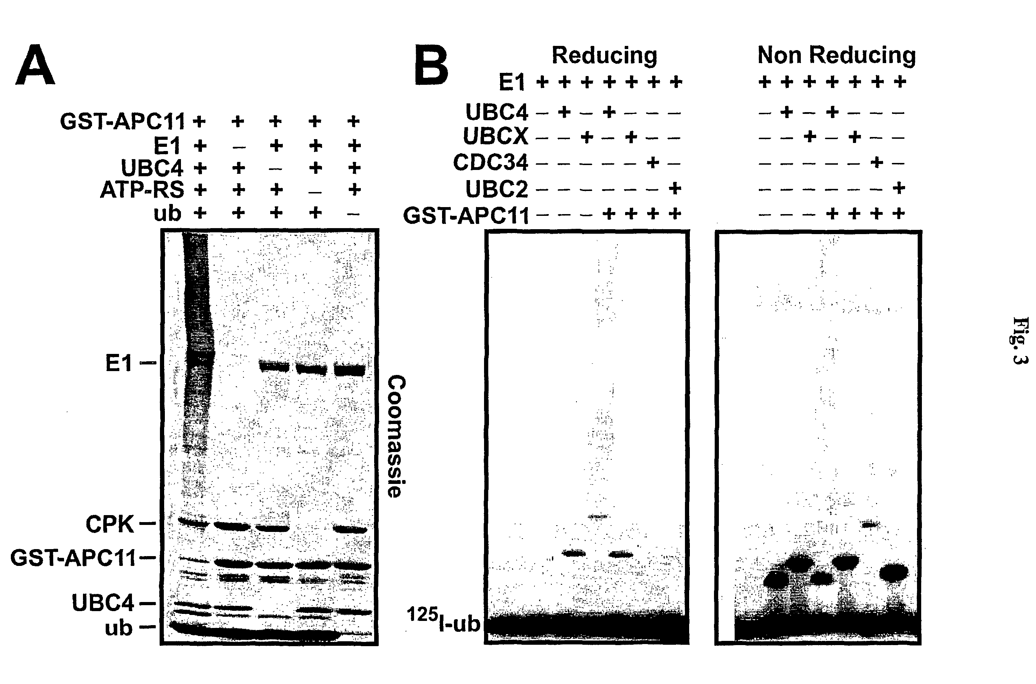

Omission of either E1, UBC4, the ATP-regenerating system, or ubiquitin from GST-APC11 containing reaction mixtures showed that the formation of multiubiquitin chains was strictly dependent on all of the above-listed components (Fig. 3A). This reaction is therefore due to a genuine E1- and E2-dependent ubiquitination reaction. APC-dependent ubiquitination reactions are supported by UBC4 and UBCx but not by other E2 enzymes (Yu, et al, 1996; Aristarkov, et al., 1996; King, et al., 1995). Likewise, it was found that reactions occurring in the presence of GST-APC11 are not supported by UBC2 or CDC34, although these enzymes were active as judged by their ability to form thioesters with ubiquitin (Fig. 3B). Unexpectedly, also UBCx was unable to support GST-APC11-dependent ubiquitination reactions (Fig. 3B).

Fig. 3:(A) Coomassie stained gel of reactions containing (+) or missing (-) the indicated components. All reactions were incubated for 30 min at 37°C, missing components were substituted with buffer. (B) Autoradiogramm of reactions separated under reducing (left) or non reducing (right) conditions. All reactions were performed in the presence of E1, ATP, ¹²⁵I-ubiquitin and with either UBC4, UBCx, CDC34, UBC2, GST-APC11, or combinations of these. Reactions were incubated for 5 min at room temperature and stopped by addition of SDS-sample buffer with or without DTT and analysed by SDS-PAGE.

### Example 4

The formation of multi-ubiquitin chains depends on the integrity of the APC11 RING-H2 finger.

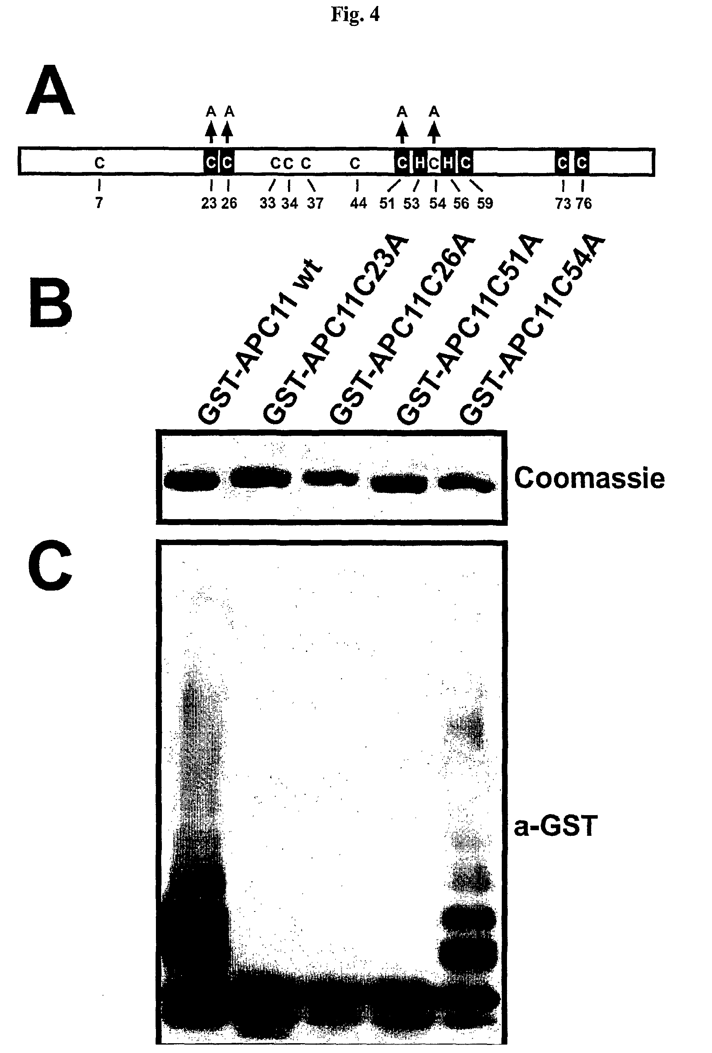

Based on analogy with RING fingers whose atomic structure is known (Borden et al., 1996), the sequence of APC11 is predicted to coordinate two atoms of zinc. It was found that ubiquitination reactions did not occur in the presence of APC11 mutants in which conserved cysteine residues of the RING-H2 finger motif are changed to alanine, whereas mutation of a cysteine residue outside the RING-H2 finger consensus had no effect (Fig. 4). The integrity of the RING-H2 finger therefore appears to be required for APC11 to support the formation of multiubiquitin chains.

(A) Schematic representation of APC11. Cysteins [C] and two histidine residues [H] are indicated. Boxed residues represent the predicted Zn coordinating amino acids. The cysteine residues that were changed to alanine [A] in this study are indicated by arrows. (B) Bacterial lysates containing GST-APC11 wild type and the indicated GST-APC11 mutants were immobilized on glutathione-beads, washed and eluted with SDS-sample buffer. Shown is a 12% polyacrylamid gel stained with Coomassie Blue. (C) Identical amounts of bacterial lysates as in (B) were bound to glutathione beads, washed and incubated with a reaction mixture as in Fig. 1 B for 30 min at 37°C. Proteins were visualized by immunodecoration with anti-GST antibodies followed by enhanced chemoluminescence.

### Example 5

GST-APC11 mediates the ubiquitination of the APC substrate Securin.

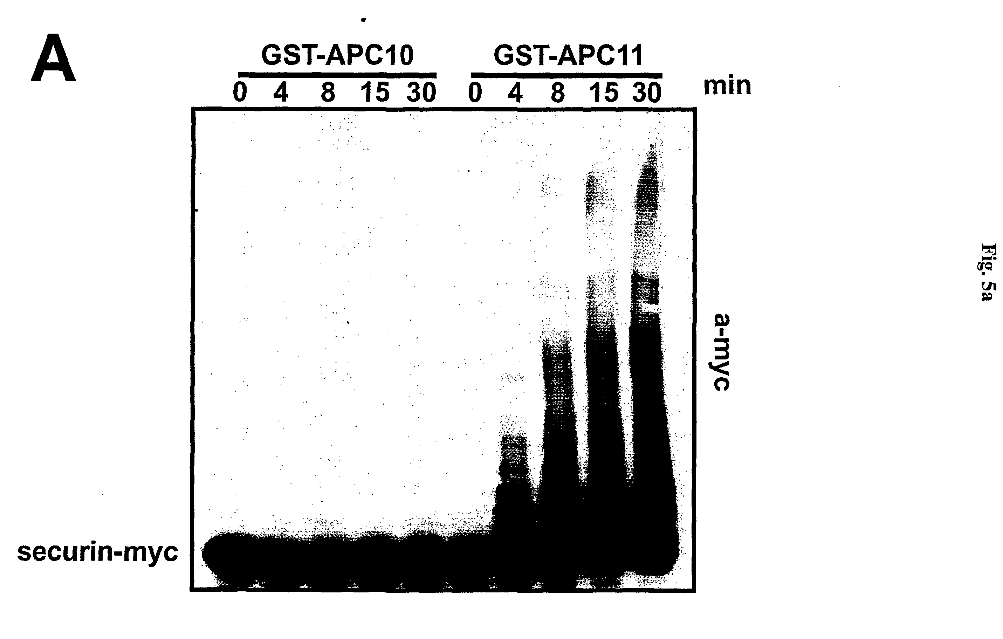

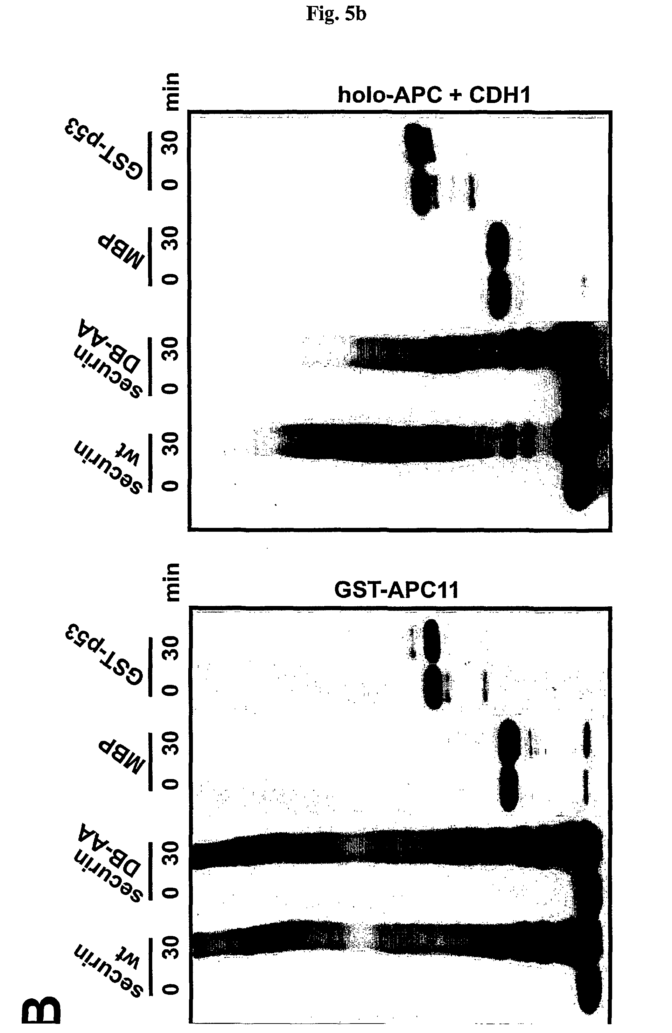

To test whether bona fide substrates of the APC can be ubiquitinated by APC11 and UBC4 purified myc-tagged human Securin was added to APC11 reaction mixtures. Securin was rapidly multiubiquitinated in reactions containing GST-APC11, whereas no ubiquitination was observed in the presence of other APC subunits (Fig. A). APC11 could also ubiquitinate cyclin B (data not shown) but not MBP and only very poorly GST-p53 (Fig. 5B). A D box mutant of Securin was ubiquitinated similarly well as wildtype Securin in the presence of GST-APC11, whereas holo-APC bound to CDH1 showed decreased activity towards the D box mutant (Fig. 5B).

(A) Time course of reactions containing either purified GST-APC10 (left) or GST-APC11 (right), E1 and UBC4, an ATP regenerating system, ubiquitin and Securin-myc-His. At the indicated time points reactions were stopped by addition of SDS-sample buffer. Separation on 15 + 8% SDS-PAGE was followed by western blotting and immunodecoration with an anti-myc antibody (9E10). (B) Purified GST-APC11 (left) or holo-APC (right) were incubated with E1, UBC4, ATP and ubiquitin as in (A) for 0 and 30 min at 37°C in the presence of either 20 ng Securin-myc-His wild type, 20 ng of Securin-myc-His D box mutant, 50 ng MBP or 50 ng of GST-p53. After separation on SDS-PAGE and transfer to PVDF membranes, proteins were detected using anti-myc (9E10) antibodies (Securin), anti-MBP antibodies or anti p53 antibodies.

### Example 6

Defining the parameters for a fluorescence resonance energy transfer (FRET) assay based on the GST-APC11-mediated formation of multiubiquitin chains

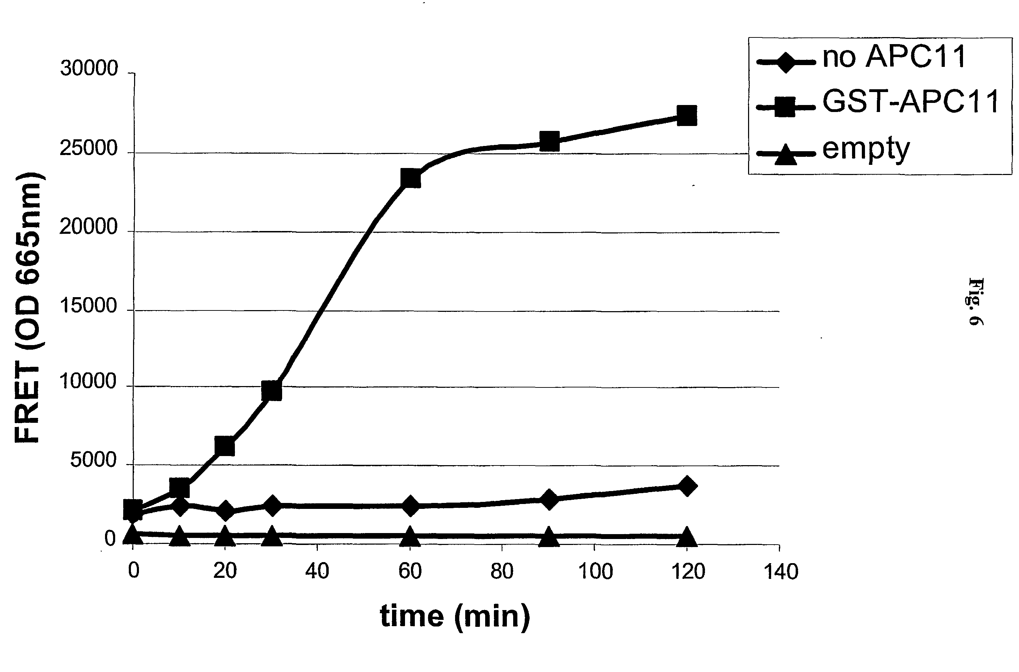

A ubiquitination reaction mixture containing the following components was assembled: 50mM Tris/HCl pH8.0, 100mM KCI, 1mM MgCl₂, 0.1mM CaCl₂, 0.5mM ATP, 1µg HIS-3xmyc-ubiquitin, 1µg biotinylated ubiquitin, 1µg GST-APC11, 0.3µg UBA1, 0.3µg HIS-UBCH5b and H₂O in a final volume of 20µl. For a control reaction GST-APC11 was omitted and substituted with H₂O. These reactions were incubated at 37°C for 0, 10, 20, 30, 60 and 120 min. At each time point a 2 µl aliquot was removed and stored on ice. To create a FRET signal, 0.5µg allophycocyanin-labeled streptavidin and 0.5µg europium labeled anti-myc antibody (both available from Wallac, Turku, Finland) were added to each aliquot. After incubation for 10 min on ice, 50µl TBST (Tris buffered saline containing Tween20) was added and the reactions transferred to a 96 well plate. The FRET signal was measured by exciting europium fluorescence at 320nm and measurement of allophycocyanin emission at 665nm in a Victor² fluorescence reader plate (Wallac, Turku, Finland) using appropriate filter settings. An example of such a kinetic study is shown in Fig. 6. As can be seen from Fig. 6, the signal obtained in the assay is approximately tenfold above background level (reaction without APC11) and the reaction is, under the chosen conditions, completed after 60 min.

Fig. 6 shows the kinetic analysis of GST-APC11-mediated formation of multiubiquitin chains.

### Example 7

Inhibition of APC11-mediated formation of multiubiquitin chains by the reducing agent DTT.

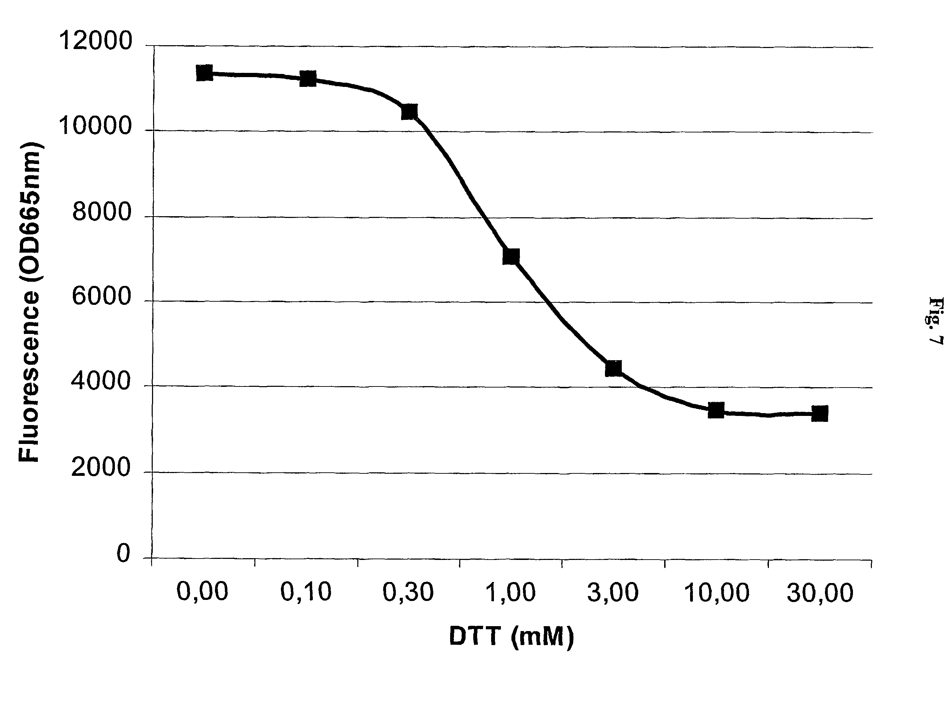

Ubiquitination reactions are dependent on the formation of thioesters between ubiquitin and the ubiquitin activating and the ubiquitin conjugating enzymes, respectively. DTT was chosen as test agent because it was expected to interfere with these reactions. DTT was titrated into the reaction mixture in a concentration range of 0.1 to 30 mM. For the particular experiment shown in Fig. 7, APC11 fused to Maltose Binding Protein (MBP) was chosen, because it was found that high expression levels of this fusion protein can be obtained in *E. coli*. In order to obtain a fully active enzyme, bacteria containing the expression plasmid for MBP-APC11 (obtained by inserting the APC11 coding region into the commercially available vector pMAL, New England Biolabs) were grown in the presence of 1 mM ZnCl₂ to ensure a sufficient amount of Zinc²⁺ ions in the medium for incorporation into the RING H2 finger of APC11. The fusion protein was purified according to standard conditions using amylose resin for affinity purification of the protein. The reaction exemplified in Fig. 7 shows the result of a ubiquitination reaction performed in the presence of 50mM Tris/HCl pH8.0, 100mM KCI, 1mM MgCl₂, 0.1mM CaCl₂, 0.5mM ATP, 0.25µg HIS-3xmyc-ubiquitin, 0.25µg biotinylated ubiquitin, 0.5µg MBP-APC11, 0.2µg UBA1, 0.3µg HIS-UBCH5b and H₂O in a final volume of 4.5µl. To each reaction 0.5µl of a serial dilution of DTT in H₂O or H₂O alone were added, resulting in final concentrations of DTT from 0 to 30mM. After incubation for 60 min at 37°C, reactions were put on ice. In order to avoid any side effects of high DTT concentrations on the fluorophore-coupled antibody, 1 µl of each reaction was diluted into 30 µl TBST before addition of 0.2 µg Eu anti myc antibodies and allophycocyanin labeled streptavidin. The result of such experiment is shown in Fig. 7.

Fig. 7 shows the inhibition of the APC11-mediated formation of multiubiquitin chains exemplified by the reducing agent DTT.

### Example 8

### High throughput assay to identify compounds that inhibit APC11-mediated formation of multiubiquitin chains

In order to perform a high throughput assay, the reaction conditions defined according to or similar to Examples 6 or 7 are adapted to microtiter plates, e.g. 384 well plates. A reaction volume of 30 µl containing all components necessary for creating a sufficiently high FRET signal (e.g. a signal at least fivefold above background), are mixed, omitting ATP as the necessary energy source. Test compounds in DMSO are added to a concentration of 10µM. The enzymatic reaction is started by addition of ATP, then the reaction mixture is incubated at 37°C for a time that is sufficient for the formation of multiubiquitin chains, usually 60min. The fluorophors (europium anti-myc antibody and allophycocyanin-labeled streptavidin) are added in quantities sufficient to generate a signal, e.g. in amounts as described in Example 6 or 7, and the resulting light emission at 665nm is recorded. Reactions containing only the solvent (DMSO) are used as positive control, reactions without ATP are employed as negative control. Compounds resulting in a significant inhibition (e.g. an inhibition of at least 50%) of the multiubiquitin chain formation are further pursued.

### References

Aristarkhov, A., Eytan, E., Moghe, A., Admon, A., Hershko, A. & Ruderman, J. V. (1996) Proc Natl Acad Sci U S A 93, 4294-9.
Borden, K. L. & Freemont, P. S. (1996) Curr Opin Struct Biol 6, 395-401.
Borden, K. L., Lally, J. M., Martin, S. R., O'Reilly, N. J., Solomon, E. & Freemont, P. S. (1996) Proc Natl Acad Sci U S A 93, 1601-6
Deshaies, R. J. (1999) Annu Rev Cell Dev Biol 15, 435-67.
Fang, S., Jensen, J. P., Ludwig, R. L., Vousden, K. H. & Weissman, A. M. (2000) J Biol Chem 275, 8945-51.
Gershkovich, A.A. and Kholodovych, V.V. (1996), J Biochem Biophys Meth 33, 135
Gieffers, C., Schleiffer, A. & Peters, J. M. (2000) Protoplasma 211, 20-28.
Grossberger R, Gieffers C, Zachariae W, Podtelejnikov AV, Schleiffer A, Nasmyth K, Mann M & Peters JM (1999) J Biol Chem **274**, 14500-7
Hatfield PM, Callis J & Vierstra RD (1990) J Biol Chem **265**, 15813-7
Hatfield PM & Vierstra RD (1992) Biol Chem **267**, 14799-803
Honda, R. & Yasuda, H. (2000) Oncogene 19, 1473-6.
Joazeiro, C. A., Wing, S. S., Huang, H., Leverson, J. D., Hunter, T. & Liu, Y.C. (1999) Science 286, 309-12.
Joazeiro CA, and Weissman AM. (2000) Cell 102, 549-52
Kamura T, Koepp DM, Conrad MN, Skowyra D, Moreland RJ, Iliopoulos O, Lane WS, Kaelin WG Jr., Elledge SJ, Conaway RC, Harper JW & Conaway J.W. (1999) Science 284, 657-61
King, R. W., Peters, J. M., Tugendreich, S., Rolfe, M., Hieter, P. & Kirschner, M. W. (1995) Cell 81, 279-88.
Kramer, E. R., Gieffers, C., Holzl, G., Hengstschlager, M. & Peters, J. M. (1998) Curr Biol 8, 1207-10.
Leverson, J.D., Joazeiro, C.A., Page, A.M., Huang, H.-k., Hieter, P., and Hunter, T. (2000) Mol. Biol. Cell 11, 2315-2325.
Levkowitz, G., Waterman, H., Ettenberg, S. A., Katz, M., Tsygankov, A. Y., Alroy, I., Lavi, S., Iwai, K., Reiss, Y., Ciechanover, A., Lipkowitz, S. & Yarden, Y. (1999) Mol Cell 4, 1029-40.
Matayoshi, E.D., Wang GT, Krafft GA, Erickson J. (1990), Science Feb 23; 247(4945):954-8
Morgan, D. O. (1999) Nature Cell Biology 1, E47-E53.
Murray, A. (1991) Methods Cell Biol. 36, 581-605.
Ohta, T., Michel, J. J., Schottelius, A. J. & Xiong, Y. (1999) Mol Cell 3, 535-41.
Peters, J.-M. (1999) Exp. Cell Res. 248, 339-349.
Pfleger, C. M. & Kirschner, M. W. (2000) Genes Dev 14, 655-65.
Pines, J. & Hunter, T. (1989) Cell 58, 833-846
Remington's Pharmaceutical Sciences, 1980, Mack Publ. Co. Easton, PA, Osol (ed.)
Selvin, (2000) Nature Structural Biol. 7(9), 730-734
Seol, J. H., Feldman, R. M., Zachariae, W., Shevchenko, A., Correll, C. C., Lyapina, S., Chi, Y., Galova, M., Claypool, J., Sandmeyer, S., Nasmyth, K. & Deshaies, R. J. (1999) Genes Dev 13, 1614-26.
Skowyra, D., Koepp, D. M., Kamura, T., Conrad, M. N., Conaway, R. C., Conaway, J. W., Elledge, S. J. & Harper, J. W. (1999) Science 284, 662-5.
Tan, P., Fuchs, S. Y., Chen, A., Wu, K., Gomez, C., Ronai, Z. & Pan, Z. Q. (1999) Mol Cell 3, 527-33.
Tugendreich S, Boguski MS, Seldin MS & Hieter P (1993) Proc Natl Acad Sci U S A 90, 10031-5
Tugendreich S, Tomkiel J, Earnshaw W & Hieter P (1995) Cell 81, 261-8
Tyers, M. & Jorgensen, P. (2000) Curr Opin Genet Dev 10, 54-64.
Xie, Y. & Varshavsky, A. (1999) Embo J 18, 6832-44.
Yokouchi, M., Kondo, T., Houghton, A., Bartkiewicz, M., Horne, W. C., Zhang, H., Yoshimura, A. & Baron, R. (1999) J Biol Chem 274, 31707-12.
Yu, H., King, R. W., Peters, J. M. & Kirschner, M. W. (1996) Curr Biol 6, 455-66.
Yu, H., Peters, J. M., King, R. W., Page, A. M., Hieter, P. & Kirschner, M. W. (1998) Science 279, 1219-22.
Zachariae, W., Shevchenko, A., Andrews, P. D., Ciosk, R., Galova, M., Stark, M. J., Mann, M. & Nasmyth, K. (1998) Science 279, 1216-9.
Zachariae, W. & Nasmyth, K. (1999) Genes Dev 13, 2039-58.
Zou H, McGarry TJ, Bernal T & Kirschner MW (1999) Science 285, 418-22

## Claims

1. A method for identifying a compound that inhibits the ubiquitination reaction mediated by the Anaphase Promoting Complex (APC), **characterized in that** a compound's ability to interfere with the formation of multiubiquitin chains by APC11 is determined.

2. The method of claim 1, **characterized in that** APC11 is incubated, together with a ubiquitin activating enzyme (E1), a ubiquitin conjugating enzyme (E2), ubiquitin and ATP for a period of time sufficient to obtain a measurable level of ubiquitination of APC11, and comparing the level of ubiquitination of APC11 in the presence or absence of a test compound.

3. The method of claim 1, **characterized in that** APC11 is incubated, together with a ubiquitin activating enzyme (E1), a ubiquitin conjugating enzyme (E2), ubiquitin and ATP for a period of time sufficient to obtain a measurable level of multiubiquitin chains, and comparing the level of multiubiquitin chains in the presence or absence of a test compound.

4. The method of claim 1, **characterized in that** APC11 is incubated, together with a ubiquitin activating enzyme (E1), a ubiquitin conjugating enzyme (E2), ubiquitin, ATP and an APC substrate protein for a period of time sufficient to obtain a measurable level of ubiquitination of the substrate protein and comparing the level of ubiquitination of the APC substrate protein in the presence or absence of a test compound.

5. The method of of any one of claims 2 to 4, wherein APC11 is human.

6. The method of of any one of claims 2 to 5, wherein E2 is the human variant UBCH5b.

7. The method of any one of claims 1 to 6, wherein E1 is wheat UBA1.

8. The method of claim 4, wherein the APC substrate protein is CyclinB.

9. The method of claim 4, wherein the APC substrate protein is Securin.

## Patentansprüche

1. Verfahren zur Identifizierung einer Verbindung, welche die durch den Anaphase-Promoting-Komplex (APC) vermittelte Ubiquitinierungsreaktion inhibiert, **dadurch gekennzeichnet, dass** die Fähigkeit einer Verbindung, die Bildung von Multiubiquitin-Ketten durch APC11 zu beeinflussen, bestimmt wird.

2. Verfahren nach Anspruch 1, **dadurch gekennzeichnet, dass** APC11 zusammen mit einem Ubiquitin-aktivierenden Enzym (E1), einem Ubiquitinkonjugierenden Enzym (E2), Ubiquitin und ATP ausreichende Zeit, um einen messbaren Ubiquitinierungsgrad von APC11 zu erhalten, inkubiert wird und der Ubiquitinierungsgrad von APC11 in Gegenwart oder Abwesenheit der Testverbindung verglichen wird.

3. Verfahren nach Anspruch 1, **dadurch gekennzeichnet, dass** APC11 zusammen mit einem Ubiquitin-aktivierenden Enzym (E1), einem Ubiquitinkonjugierenden Enzym (E2), Ubiquitin und ATP ausreichende Zeit, um einen messbaren Gehalt an Multiubiquitin-Ketten zu erhalten, inkubiert wird und der Gehalt an Multiubiquitin-Ketten in Gegenwart oder Abwesenheit der Testverbindung verglichen wird.

4. Verfahren nach Anspruch 1, **dadurch gekennzeichnet, dass** APC11 zusammen mit einem Ubiquitin-aktivierenden Enzym (E1), einem Ubiquitinkonjugierenden Enzym (E2), Ubiquitin und ATP ausreichende Zeit, um einen messbaren Ubiquitinierungsgrad des Substratproteins zu erhalten, inkubiert wird und der Ubiquitinierungsgrad des APC-Substratproteins in Gegenwart oder Abwesenheit der Testverbindung verglichen wird.

5. Verfahren nach irgendeinem der Ansprüche 2 bis 4, wobei das APC11 human ist.

6. Verfahren nach irgendeinem der Ansprüche 2 bis 5, wobei E2 die Humanvariante UBCH5b ist.

7. Verfahren nach irgendeinem der Ansprüche 2 bis 6, wobei E1 Weizen-UBA1 ist.

8. Verfahren nach Anspruch 4, wobei das APC-Substratprotein CyclinB ist.

9. Verfahren nach Anspruch 4, wobei das APC-Substratprotein Securin ist.

## Revendications

1. Procédé pour identifier un composé qui inhibe la réaction d'ubiquitination à médiation par le complexe promoteur de l'anaphase (APC), **caractérisé en ce que** l'aptitude d'un composé à interférer avec la formation de chaînes de multiubiquitine par APC11 est déterminée.

2. Procédé selon la revendication 1 **caractérisé en ce que** APC11 est incubé, en même temps qu'une enzyme activant l'ubiquitine (E1), une enzyme conjuguant l'ubiquitine (E2), l'ubiquitine et l'ATP pendant une durée suffisante pour obtenir un niveau mesurable d'ubiquitination d'APC11, et le niveau d'ubiquitination d'APC11 en présence ou en l'absence d'un composé à tester est comparé.

3. Procédé selon la revendication 1 **caractérisé en ce que** APC11 est incubé, en même temps qu'une enzyme activant l'ubiquitine (E1), une enzyme conjuguant l'ubiquitine (E2), l'ubiquitine et l'ATP pendant une durée suffisante pour obtenir un niveau mesurable de chaînes de multiubiquitine, et le niveau de chaînes de multiubiquitine en présence ou en l'absence d'un composé à tester est comparé.

4. Procédé selon la revendication 1 **caractérisé en ce que** APC11 est incubé, en même temps qu'une enzyme activant l'ubiquitine (E1), une enzyme conjuguant l'ubiquitine (E2), l'ubiquitine, l'ATP et une protéine substrat d'APC pendant une durée suffisante pour obtenir un niveau mesurable d'ubiquitination de la protéine substrat, et le niveau d'ubiquitination de la protéine substrat d'APC en présence ou en l'absence d'un composé à tester est comparé.

5. Procédé selon l'une quelconque des revendications 2 à 4 où APC11 est humain.

6. Procédé selon l'une quelconque des revendications 2 à 5 où E2 est le variant humain UBCH5b.

7. Procédé selon l'une quelconque des revendications 1 à 6 où E1 est UBA1 de froment.

8. Procédé selon la revendication 4 où la protéine substrat d'APC est la cycline B.

9. Procédé selon la revendication 4 où la protéine substrat d'APC est la sécurine.
